# EUROPEAN PATENT APPLICATION

(11) **EP 3 954 785 A1**
(43) Date of publication of application: **16.02.2022**
(21) Application number: 21771650.5
(22) Date of filing: 10.03.2021
(51) Int. Cl.: C12Q 1/70, G01N 33/543

(54) **LATERAL FLOW ANALYSIS STRIP AND MOLECULAR DIAGNOSTIC METHOD USING SAME**

(30) Priority: 17.03.2020 KR 20200032435; 08.05.2020 KR 20200055330
(71) Applicant: PHILMEDI Co., Ltd., Gyeonggi-do 13120 (KR)
(72) Inventor: LEE, Byung Hwan, Yongin-si Gyeonggi-do 17086 (KR); KIM, Se Jin, Seongnam-si Gyeonggi-do 13279 (KR); KIM, Dami, Seongnam-si Gyeonggi-do 13162 (KR)
(74) Representative: Stolmár & Partner Patentanwälte PartG mbB
(86) International application number: PCT/KR2021/002898
(87) International publication number: WO 2021/187791

(57) **Abstract**

Disclosed are a lateral flow analysis strip and a molecular diagnostic method using same. The lateral flow analysis strip may comprise: a sample pad (100) into which a sample including at least one of a tag-labeled amplicon and a tag-labeled amplicon precursor is to be introduced; a conjugate pad (200) including a conjugate (C1) which has an indicator and a detector bound to the surface of the indicator and to which the conjugate (C1) is non-fixedly adsorbed; a test pad (300) including a test line (310) to which a first captor is fixed; a control pad (400) including a control line (410) to which a second captor is fixed; and an absorption pad (500). The lateral flow analysis strip of the present invention can detect an amplicon, such as a nucleic acid, amplified using an amplicon precursor such as a primer or dNTP, labeled with one type of tag, and can directly determine a result with the naked eye. In addition, the lateral flow analysis strip advantageously has a high sensitivity by using a tag having a strong binding force, a captor binding to the tag, a detector, and a captor binding to the detector. Further, the lateral flow analysis strip has a reproducible and stable effect by using a tag having a certain binding force, a captor binding to the tag, a detector, and a captor binding to the detector.

## Description

### Technical Field

The present disclosure relates to a lateral flow assay strip and a molecular diagnostic method using the same. More particularly, the present disclosure relates to a lateral flow assay strip capable of detecting an amplicon which is amplified using an amplicon precursor labelled with one type of tag and of performing a diagnosis, and a molecular diagnostic method using the same lateral flow assay strip.

### Background Art

As a conventional method of detecting and diagnosing viruses or nucleic acids, methods using an electron microscope or serological methods have been mainly used. Although methods using an electron microscope can confirm presence of viruses, it is almost impossible to identify a species by its morphological characteristics. Among serological methods, an enzyme-linked immunosorbent assay (ELISA) is the most common but it has about 1,000 times lower detection sensitivity than a polymerase chain reaction (PCR) diagnostic method and often fails to carry out an accurate diagnosis due to unexpected nonspecific reactions.

Recently, a PCR method having a high detection sensitivity and convenience has been used to detect viruses or nucleic acids and to perform a diagnosis. However, the PCR- based diagnosis has problems in that it requires the development of a specific primer and involves a series of steps of confirming amplified reaction products by electrophoresis and performing DNA sequencing. In addition, the method also requires specialized equipment such as a polymerase chain reaction device (thermocycler) and professional technicians capable of operating the device. The DNA sequencing to confirm final amplification products is a process that requires much money and high-end technology. Furthermore, it takes long time to carry out the series of steps described above, and the results of the diagnosis cannot be confirmed with the naked eye. Therefore, there is a problem in that it is difficult to use the method in the environment in which specific analysis equipment lacks.

Therefore, to effectively detect viruses or genes within a short time, it is required to develop diagnostic equipment and method enabling the detection of viruses or genes in real time in the field not equipped with specialized equipment.

### Disclosure

### Technical Problem

Accordingly, the present disclosure has been made keeping in mind the above problems occurring in the related art, and an objective of the present disclosure is to provide a lateral flow assay strip capable of detecting an amplicon such as a nucleic acid that is amplified using a primer which is labelled with one type of tag, or a precursor such as dNTP, and confirming a result immediately with naked eyes.

Another objective of the present disclosure is to provide a lateral flow assay strip which has a high sensitivity and uses at least one tag, a trapping molecule bound to the tag(s), a detector, and a trapping molecule bound to the detector, and a molecular diagnostic method using the same.

A further objective of the present disclosure is to provide a reproducible and stable lateral flow assay strip which uses at least one tag having a constant binding force, a trapping molecule bound to the tag(s), a detector, and a trapping molecule bound to the detector, and a molecular diagnostic method using the same.

### Technical Solution

According to one aspect of the present disclosure, a lateral flow assay strip 10 includes: a sample pad 100 configured to receive a sample containing at least one of amplicons labelled with at least one tag and amplicon precursors labelled with a tag; a conjugate pad 200 which includes a first conjugate C1 having an indicator and a detector bound to the indicator, with the first conjugate C1 attached in a flowable manner; a test pad 300 which includes a test line 310 with a first trapping molecule fixed; a control pad 400 which includes a control line 410 with a second trapping molecule fixed; and an absorption pad 500.

In addition, the detector may be the same type as the first trapping molecule.

In addition, each of the detector and the first trapping molecule may include one selected from a group consisting of an avidin and an anti-biotin antibody.

In addition, the indicator may include one selected from a group consisting of gold (Au), silver (Ag), copper (Cu), platinum (Pt), palladium (Pd), ruthenium (Ru), titanium (Ti), zirconium (Zr), hafnium (Hf), vanadium (V), niobium (Nb), tantalum (Ta), chromium (Cr), molybdenum (Mo), tungsten (W), osmium (Os), iron (Fe), nickel (Ni), cobalt (Co), magnesium oxide (MgO), titanium dioxide (TiO₂), vanadium pentoxide (V₂O₅), and zinc oxide (ZnO).

In addition, at least one tag labelled on the amplicon and the tag labelled on the amplicon precursor each may be the same type as the second trapping molecule.

In addition, each of the tag and the second trapping molecule may include biotins.

In addition, the amplicon labelled with the tag may be labelled with two or more tags.

In addition, the sample may include an amplicon labelled with at least one tag. The sample pad 100 may be configured to receive the sample and to discharge the sample into the conjugate pad 200. The conjugate pad 200 may be configured to bind at least one tag labelled on the amplicon, to a portion of the first conjugate C1 so that the first conjugate C1 and a second conjugate C2 that is bound to at least one tag labelled on the amplicon are discharged into a test pad 300. The test pad 300 may be configured to bind the second conjugate C2 that is bound to at least one tag labelled on the amplicon, discharged from the conjugate pad 200, to the first trapping molecule on the test line 310 to trap the second conjugate C2 and may be configured to discharge the first conjugate C1 into a control pad 400. The control pad 400 may be configured to bind the first conjugate C1 discharged from the test pad 300, to a second trapping molecule on a control line 410 to trap the first conjugate C1.

In addition, the sample includes an amplicon precursor labelled with a tag, a sample pad 100 may be configured to receive the sample and to discharge the sample to a conjugate pad 200, the conjugate pad 200 may be configured to bind at least one tag labelled on the amplicon, to a portion of the first conjugate C1 to discharge the first conjugate C1 and a third conjugate C3 that is bound to the tag labelled on the amplicon precursor to a test pad 300, the test pad 300 may be configured to discharge the first conjugate C1 discharged from the conjugate pad 200 and the third conjugate C3 bound to the tag labelled on the amplicon precursor, to a control pad 400, the control pad 400 may be configured to bind the first conjugate C1 discharged from the test pad 300, to a second trapping molecule on a control line 410 to trap the first conjugate C1, and is configured to discharge the third conjugate C3 bound to the tag labelled on the amplicon precursor, to an absorption pad 500, and the absorption pad 500 may be configured to absorb the third conjugate C3 bound to the tag labelled on the amplicon precursor.

In addition, the amplicon precursor may include at least one selected from a group consisting of dNTP, dUTP, a forward inner primer (FIP), a backward inner primer (BIP), a forward outer primer (F3), a backward outer primer (B3), a backward loop primer (LB) and a forward loop primer (LF).

Also, the amplicon labelled with at least one tag, may be amplified by loop mediated isothermal amplification (LAMP).

Furthermore, the lateral flow assay strip may be used to diagnose at least one selected from among bacterial pneumonia, tuberculosis, and gonorrhea, and diseases caused by at least one virus selected from a group consisting of influenza virus, AIDS virus (HIV), variola virus, foot-and-mouth disease virus, Ebola virus, dengue virus, Zika virus, corona virus, HPV virus.

In addition, a dissociation constant (Kd) of an association between the tag(s) and the first trapping molecule, and a dissociation constant (Kd) of an association between the detector and the second trapping molecule may be each independently 10^-16 M to 10^-14 M.

In addition, each of the sample pad 100, the conjugate pad 200, the control pad 400, and the absorption pad 500 may be disposed in this order in a horizontal direction, and the test pad 300 may be disposed between the conjugate pad 200 and the control pad 400 or may be disposed between the control pad 400 and the absorption pad 500.

According to another aspect of the present disclosure, there is provided a molecular diagnostic method that uses a lateral flow assay strip including a sample pad 100, a conjugate pad 200, a test pad 300, a control pad 400, and an absorption pad 500. The method includes steps of: (a) introducing a sample containing an amplicon labelled with at least one tag, into the sample pad 100; (b) by the sample pad 100, discharging the sample into the conjugate pad 200; (c) by the conjugate pad 200, binding at least one tag labelled on the amplicon, to a portion of a first conjugate C1, followed by discharging the first conjugate C1, and a second conjugate C2 bound to at least one tag labelled on the amplicon, into the test pad 300, wherein the conjugate pad 200 includes the first conjugate C1 having an indicator and a detector bound to a surface of the indicator, with the first conjugate C1 attached in a flowable manner; (d) by the test pad 300, binding the second conjugate C2 bound to at least one tag labelled on the amplicon discharged from the conjugate pad 200, to the first trapping molecule on the test line 310, to trap the second conjugate C2 and discharging the first conjugate C1 that is not trapped by the first trapping molecule to the control pad 400, wherein the test pad 300 includes the test line 310 having the first trapping molecule fixed; and (e) by the control pad 400, binding the first conjugate C1 discharged from the test pad 300, to the second trapping molecule on the control line 410, and trapping the first conjugate C1, wherein the control pad 400 includes the control line 410 having the second trapping molecule fixed.

In addition, the molecular diagnostic method using the lateral flow assay strip 10 may further include the step of amplifying a mixture containing a nucleic acid, an amplicon precursor which is labelled with a tag, and an amplicon precursor which is not labelled with any tag, to make an amplicon which is labelled with a tag or a plurality of tags (step a'), before step (a), wherein the amplicon precursor may include at least one selected from a group consisting of dNTP, dUTP, a forward inner primer (FIP), a backward inner primer (BIP), a forward outer primer (F3), a backward outer primer (B3), a backward loop primer (LB), and a forward loop primer (LF).

In addition, a ratio C2/C1 of the concentration C2 of the amplicon precursor labelled with a tag in the mixture to the concentration C1 of the amplicon precursor not labelled with any tag, used in step (a'), is 0.01 to 0.5.

In addition, the amplification may be performed by loop mediated isothermal amplification (LAMP), and the loop mediated isothermal amplification may be performed by maintaining a predetermined temperature via at least one selected from a group consisting of a hot pack, a hand warmer, a portable tumbler, and a portable heating device.

In addition, the amplification may be performed at a temperature of 60°C to 70°C.

In addition, the amplicon labelled with at least one tag may be labelled with two or more tags.

In addition, a molecular diagnostic method using the lateral flow assay strip 10 may further include the step of confirming whether each of the test line 310 and the control line 410 generates a colored band, followed by performing a diagnosis (step f), after step (e).

According to another aspect of the present disclosure, there is provided a molecular diagnostic method that uses the lateral flow assay strip 10 including a sample pad 100, a conjugate pad 200, a test pad 300, a control pad 400 and an absorption pad 500. The method includes the steps of: introducing a sample containing an amplicon precursor which is labelled with a tag, into the sample pad 100 (step 1); by the sample pad 100, discharging the sample to the conjugate pad 200 (step 2); by the conjugate pad 200, binding the tag which is labelled on the amplicon precursor to a portion of the first conjugate C1, followed by discharging the first conjugate C1, and the third conjugate C3 bound to the tag which is labelled on the amplicon precursor, to the test pad 300, wherein the conjugate pad 200 includes a first conjugate C1 having an indicator, and a detector bound to a surface of the indicator, with the first conjugate C1 bound in a flowable manner (step 3); by the test pad 300, discharging the first conjugate C1 discharged from the conjugate pad 200, and the third conjugate C3 bound to the tag which is labelled on the amplicon precursor, into the control pad 400, wherein the test pad 300 includes a test line 301 having a first trapping molecule fixed (step 4); by the control pad 400, binding the first conjugate C1 discharged from the test pad 300, to the second trapping molecule on the control line 310 to trap the first conjugate C1, and discharging the third conjugate C3 bound to the tag which is labelled on the amplicon precursor, to the absorption pad 500, wherein the control pad 400 includes a control line 410 having fixed a second trapping molecule fixed (step 5); and by the absorption pad 500, absorbing the third conjugate C3 bound to a tag labelled on the amplicon precursor (step 6).

In addition, the molecular diagnostic method using the lateral flow assay strip 10 may further include the step of amplifying a mixture containing an amplicon precursor which is labelled with a tag and an amplicon precursor which is not labelled with any tag (step 1'), before the step 1, wherein the amplification results are not obtained.

In addition, a molecular diagnostic method using the lateral flow assay strip 10 may further include the step of confirming that the test line 310 does not generate a colored band, and the control line 410 generate a colored band, separately, followed by performing a diagnosis (step 7), after step 6.

### Advantageous Effects

As described above, a lateral flow assay strip of the present disclosure has an effect of being able to detect an amplicon such as a nucleic acid amplified using an amplicon precursor such as dNTP, or a primer which is labelled with one type of tag and confirming a result immediately by naked eye.

In addition, a lateral flow assay strip of the present disclosure has an effect of providing a high sensitivity by using at least one tag having a strong binding force, a trapping molecule which binds to the tag(s), a detector, and a trapping molecule which binds to the detector.

In addition, the present disclosure has an effect of providing a reproducible and stable lateral flow assay strip by using at least one tag having a constant binding force, a trapping molecule which binds to the tag(s), a detector, and a trapping molecule which binds to the detector.

### Description of Drawings

FIG. 1 is a schematic diagram showing a structure of a lateral flow assay strip of the present disclosure;
FIG. 2 is a schematic diagram showing a detection path when a sample containing an amplicon labelled with at least one tag, is introduced into a lateral flow assay strip of the present disclosure;
FIG. 3 is a schematic diagram showing a detection path when a sample containing an amplicon precursor labelled with a tag, is introduced into a lateral flow assay strip of the present disclosure;
FIG. 4 is a schematic diagram showing an amplification reaction when a nucleic acid to be diagnosed is present;
FIG. 5 is a schematic diagram showing an amplification reaction when a nucleic acid to be diagnosed is absent;
FIG. 6 is LFA test results of a synthesized oligomer which is labelled with six biotins and a synthesized oligomer which is labelled with one biotin;
FIG. 7 is an image of amplification results in Example 2-2, 2-4, 2-5 and 3-2;
FIG. 8 is an image of electrophoresis showing amplification results in Example 2-2, 2-4, 2-5 and 3-2;
FIG. 9 is a lateral flow assayed result by introducing products amplified in Example 2-1 to 2-3, and 3-1 to 3-3, into a LFA strip prepared in Example 1; and
FIG. 10 is a lateral flow assayed result by introducing products amplified in Example 2-2, 2-4, 2-5 and 3-2, into a LFA strip prepared in Example 1.

### Best Mode

### Mode for Disclosure

Hereinafter, embodiments of the present disclosure will be described in detail with reference to the accompanying drawings so that those of ordinary skill in the art to which the present disclosure pertains can easily implement same.

However, the following description is not intended to limit the present disclosure to specific embodiments, and when it is determined that detailed descriptions of related known techniques may obscure the gist of the present disclosure in describing the present disclosure, the detailed description thereof will be omitted.

The terminology used herein is used only to describe specific embodiments and is not intended to limit the present disclosure. The singular expression includes the plural expression unless the context clearly dictates otherwise. In the present application, terms such as "comprise" or "have" are intended to specify that a feature, number, step, operation, element, or combination thereof described in the specification is present. It is to be understood that it does not preclude the possibility of the presence or addition of one or more other features, numbers, steps, acts, elements, or combinations thereof.

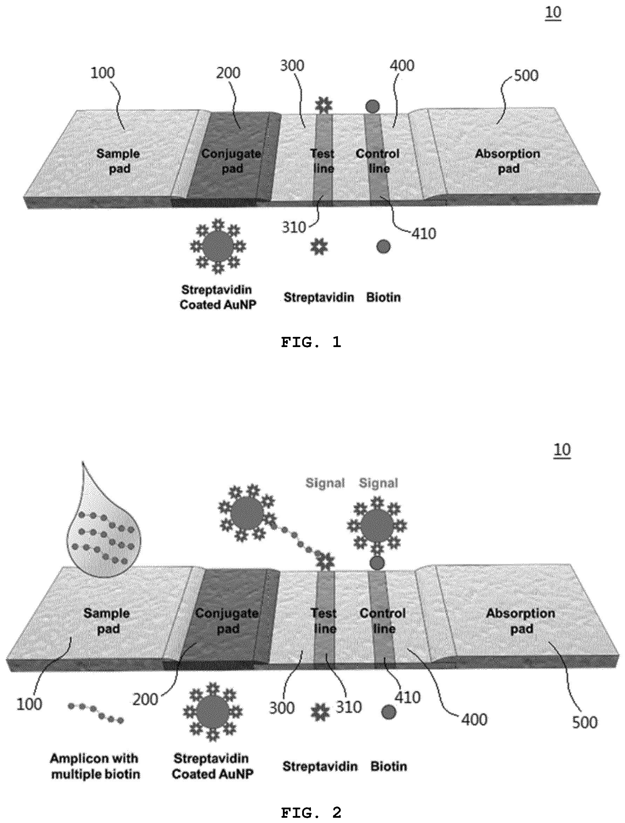

FIG. 1 is a schematic diagram showing a structure of a lateral flow assay strip 10 of the present disclosure. Hereinafter, the lateral flow assays strip 10 of the present disclosure will be described with reference to FIG. 1.

### Sample pad 100

A lateral flow assay strip 10 of the present disclosure may include a sample pad 100 configured to receive a sample containing at least one of amplicons which is labelled with at least one tag, and amplicon precursors which is labelled with a tag.

The amplicon labelled with at least one tag, may be labelled with two or more tags.

The amplicon precursor may include at least one selected from a group consisting of dNTP, dUTP, a forward inner primer (FIP), a backward inner primer (BIP), a forward outer primer (F3), a backward outer primer (B3), a backward loop primer (LB) and a forward loop primer (LF).

The amplicon labelled with at least one tag, may be amplified by Loop mediated isothermal amplification (LAMP).

### Conjugate pad 200

A lateral flow assay strip 10 of the present disclosure may include a first conjugate (C1) having an indicator and a detector which binds to a surface of the indicator, and a conjugate pad 200 having the first conjugate (C1) attached in a flowable manner.

The indicator may include one selected from a group consisting of gold (Au), silver (Ag), copper (Cu), platinum (Pt), palladium (Pd), ruthenium (Ru), titanium (Ti), zirconium (Zr), hafnium (Hf), vanadium (V), niobium (Nb), tantalum (Ta), chromium (Cr), molybdenum (Mo), tungsten (W), osmium (Os), iron (Fe), nickel (Ni), cobalt (Co), magnesium oxide (MgO), titanium dioxide (TiO₂), vanadium pentoxide (V₂O₅), and zinc oxide (ZnO), and may preferably include gold (Au).

### Test pad 300

A lateral flow assay strip 10 of the present disclosure may include a test pad 300 including a test line having a first trapping molecule fixed.

### Control pad 400

A lateral flow assay strip 10 of the present disclosure may include a control pad 400 including a control line 410 having a second trapping molecule fixed.

### Absorption pad 500

A lateral flow assay strip 10 of the present disclosure may include an absorption pad 500.

The detector may be a same type of the first trapping molecule.

Each of the detector and the first trapping molecule may include one selected from the group consisting of an avidin and an anti-biotin antibody, and preferably may include an avidin.

Each of at least one tag labelled on the amplicon and the tag which is labelled on the amplicon precursor is a same type of the second trapping molecule.

Each of the tags and the second trapping molecule may include biotins.

According to an embodiment of the present disclosure, the sample includes an amplicon which is labelled with at least one tag, the sample pad 100 may be configured to receive the sample and to discharge the sample to the conjugate pad 200, the conjugate pad 200 may be configured to bind at least one tag labelled on the amplicon, to a portion of the first conjugate C1, so that the first conjugate C1 and a second conjugate C2 bound to at least one tag labelled on the amplicon, are discharged into the test pad 300, the test pad 300 may be configured to bind the second conjugate C2 that is bound to at least one tag labelled on the amplicon discharged from the conjugate pad 200, to the first trapping molecule on the test line 310 to trap the second conjugate C2, and configured to discharge the first conjugate C1 to the control pad 400, and the control pad 400 may be configured to bind the first conjugate C1 discharged from the test pad 300, to the second trapping molecule on the control line 410 to trap the first conjugate C1.

In addition, according to another embodiment of the present disclosure, the sample pad 100 may be configured to receive the sample and to discharge the sample to a conjugate pad 200, the conjugate pad 200 may be configured to bind at least one tag labelled on the amplicon, to a portion of the first conjugate C1 to discharge the first conjugate C1 and a third conjugate C3 that is bound to the tag labelled on the amplicon precursor to a test pad 300, the test pad 300 may be configured to discharge the first conjugate C1 discharged from the conjugate pad 200 and the third conjugate C3 bound to the tag labelled on the amplicon precursor, to the control pad 400, the control pad 400 may be configured to bind the first conjugate C1 discharged from the test pad 300, to a second trapping molecule on the control line 410 to trap the first conjugate C1, and may be configured to discharge the third conjugate C3 bound to the tag labelled on the amplicon precursor, to the absorption pad 500, and the absorption pad 500 may be configured to absorb the third conjugate C3 bound to the tag labelled on the amplicon precursor.

The lateral flow assay strip may be used to diagnose at least one selected from among bacterial pneumonia, tuberculosis, and gonorrhea, and diseases caused by at least one virus selected from a group consisting of influenza virus, AIDS virus (HIV), variola virus, foot-and-mouth disease virus, Ebola virus, dengue virus, Zika virus, corona virus, HPV virus.

A dissociation constant (Kd) of an association between the tag(s) and the first trapping molecule, and a dissociation constant (Kd) of an association between the detector and the second trapping molecule, may be each independently 10^-16 M to 10^-14 M.

Each of the sample pad 100, the conjugate pad 200, the control pad 400, and the absorption pad 500 may be disposed in this order in a horizontal direction, and the test pad 300 may be disposed between the conjugate pad 200 and the control pad 400 or may be disposed between the control pad 400 and the absorption pad 500, and it is also possible to make these bind each other and be disposed in that order.

The sample pad 100, the conjugate pad 200, the test pad 300, the control pad 400, and the absorbent pad 500 may be porous and hydrophilic. So, when a sample is being introduced into a sample pad 100, the sample may move into an absorption pad 500 through the sample pad 100, the conjugate pad 200, the test pad 300, the control pad 400. In this case, some of sample compositions may bind to a trapping molecule which is present in at least one of the pads, and then be captured on a surface of at least one of the pads.

FIG. 2 is a schematic diagram showing a detection path when a sample containing an amplicon labelled with at least one tag, is introduced into a lateral flow assay strip of the present disclosure.

Hereinafter, a molecular diagnostic method using a lateral flow assay strip 10 according to an embodiment of the present disclosure will be described with reference to FIG. 2.

First, a sample containing an amplicon which is labelled with at least one tag is introduced into the sample pad 100 (step a).

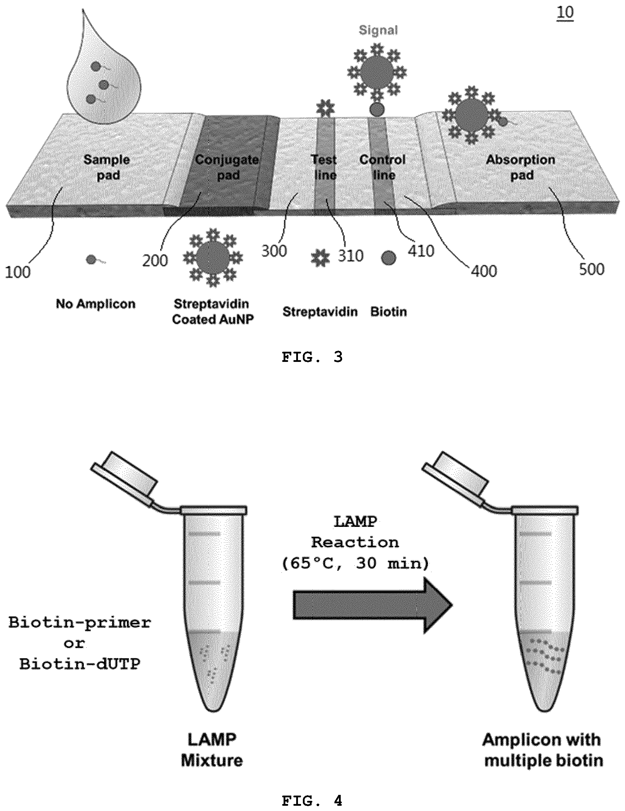

The present method may further include a step of amplifying a mixture containing a nucleic acid, an amplicon precursor which is labelled with a tag, and an amplicon precursor which is not labelled with any tag, to make an amplicon which is labelled with a tag or a plurality of tags (step a'), before step a, wherein the amplicon precursor may include at least one selected from a group consisting of dNTP, dUTP, a forward inner primer (FIP), a backward inner primer (BIP), a forward outer primer (F3), a backward outer primer (B3), a backward loop primer (LB), and a forward loop primer (LF). FIG. 4 shows an amplification reaction of step (a').

In a mixture of step (a'), a ratio C2/C1 of a concentration C2 of the amplicon precursor labelled with a tag in the mixture to a concentration C1 of the amplicon precursor not labelled with any tag may be 0.01 to 0.5, preferably may be 0.03 to 0.3, and more preferably may be 0.07.

A concentration of the amplicon precursor which is not labelled with any tag in step (a') may be 0.1 mM to 1.5 mM, preferably may be 0.2 mM to 0.5 mM, and more preferably may be 0.3 mM. When the concentration of the amplicon precursor which is not labelled with any tag is less than 0.1 mM, a signal strength of a test line is lowered, so it not preferable. When the concentration of the amplicon precursor which is not labelled with any tag is more than 1.5 mM, an effect of an amplicon precursor which is added is insignificant, so it not preferable.

A concentration of the amplicon precursor which is labelled with a tag in step (a') may be 0.01 mM to 0.1 mM, preferably may be 0.01 mM to 0.03 mM, and more preferably may be 0.02 mM. When the concentration of the amplicon precursor which is labelled with a tag is less than 0.01 mM, a concentration of an amplicon precursor which is not labelled with any tag, to a concentration of an amplicon precursor which is labelled with a tag is relatively high, resulting in lowering a signal strength of a test line, so it not preferable. When the concentration of the amplicon precursor which is labelled with a tag is more than 0.1 mM, a signal strength is lowered, so it not preferable.

The amplification is performed by Loop mediated isothermal amplification (LAMP), and the loop mediated isothermal amplification may be performed by maintaining a predetermined temperature via at least one selected from the group consisting of a hot pack, a hand warmer, a portable tumbler, and a portable heating device.

The isothermal amplification may be performed at a temperature of 60°C to 70°C, at which an activity of an enzyme used is not substantially suppressed, preferably may be performed at a temperature of 65°C. When the temperature is less than 60°C or more than 70°C, an activity of Bst polymerase decreases drastically, so it is not preferable. Particularly, a portable device capable of maintaining a predetermined temperature may be used, because a reaction of LAMP proceeds in an isothermal condition. Particularly, a portable tumbler, a portable heating device, a portable electric heating device may also be used, and a portable heating source which accommodates a steel in a closed space and generates heat through oxidation of the steel, may also be used. Particularly, a commercially available hot pack and hand warmer, which may maintain a temperature of 60°C to 70°C, at which an isothermal amplification reaction may proceed, may also be used. In the future, LAMP, in which the portable devices may be used, can be applied as a simple diagnostic means in front-line clinical diagnostic lab or clinical fields without expensive special equipment.

The amplicon which is labelled with at least one tag may be labelled with two or more tags.

Next, the sample is discharged to the conjugate pad 200 by the sample pad 100 (step b).

Next, at least one tag labelled on the amplicon binds to a portion of the first conjugate C1, and then the first conjugate C1 and the second conjugate C2 bound to at least one tag labelled on the amplicon is discharged to the test pad 300, by the conjugate pad 200 (step c).

Next, the second conjugate C2 bound to at least one tag labelled on the amplicon discharged from the conjugate pad 200 binds to the first trapping molecule on the test line 310 to trap the second conjugate C2, and the first conjugate C1 which is not captured by the first trapping molecule is discharged to the control pad 400, by the test pad 300 (step d) .

Finally, the first conjugate C1 discharged from the test pad 300, binds to a second trapping molecule on the control line 410 and is captured thereon, by the control pad 400 (step e).

A method of the present disclosure may further include step of confirming that each of the test line 310 and the control line 410 generates a colored band to make a diagnosis (step f), after step (e).

FIG. 3 is a schematic diagram showing a detection path when a sample containing an amplicon precursor labelled with a tag, is introduced into a lateral flow assay strip of the present disclosure.

Hereinafter, a molecular diagnosis method using a lateral flow assay strip 10 according to another embodiment of the present disclosure will be described with reference to FIG. 3.

First, a sample containing an amplicon precursor which is labelled with a tag is introduced into the sample pad 100 (step 1).

A present method using a lateral flow assay strip 10 may further include a step of amplifying a mixture containing an amplicon precursor which is labelled with a tag and an amplicon precursor which is not labelled with any tag (step 1'), before the step 1, wherein the amplification results are not obtained.

Next, the sample is discharged to the conjugate pad 200 by the sample pad 100 (step 2).

Next, the tag which is labelled on the amplicon precursor binds to a portion of the first conjugate C1, and then the first conjugate C1, and the third conjugate C3 bound to the tag which is labelled on the amplicon precursor is discharged to the test pad 300, by the conjugate pad 200 (step 3) .

Next, the first conjugate C1 discharged from the conjugate pad 200, and the third conjugate C3 bound to a tag labelled on the amplicon precursor, is discharged to the control pad 400, by the test pad 300 (step 4).

Next, the first conjugate C1 discharged from the test pad 300 binds to a second trapping molecule on the control line 310 to trap the first conjugate C1, and discharges the third conjugate C3 bound to the tag labelled on the amplicon precursor, to the absorption pad 500, by the control pad 400 (step 5).

Finally, the absorption pad 500 absorbs the third conjugate C3 bound to a tag labelled on the amplicon precursor (step 6).

The method of the present disclosure may further include a step of confirming that the test line 310 does not generate a colored band and the control line 410 generate a colored band, followed by performing a diagnosis (step 7), after step 6.

### Example

Hereinafter, the present disclosure will be described in more detail by way of examples. However, this is for illustrative purposes, and the scope of the present disclosure is not limited thereto.

### Preparation example 1: Preparation of streptavidin-AuNP conjugation solution

1 mL of 40 nm AuNP (Cat. EM. GC40, BBI Solutions) solution, 100 µL of borate buffer (pH 8.5, 0.1 M, Cat. BB001, Bio-solution) and a streptavidin (Cat. 434302, Thermo) was mixed to a final concentration of 10 µg/mL to prepare a mixed solution. The mixture solution was vortexed and spun down, followed by performing incubation for 1 hour at an ambient temperature. 10 µL of PBS (10 mM, pH 7.4) containing 100 mg/mL of BSA was added to the mixture solution to a final concentration of 0.1% to block a surface of AuNP. This mixture was vortexed and spun down, followed by performing incubation for 2 hours at an ambient temperature. The mixture was centrifuged by using a centrifuge at 9,000 rpm and 10°C for 15 minutes. The supernatant was discarded, and 1 mL of borate buffer (10 mM, pH 8.5) was added and perform suspension. After repeating the centrifugation and suspension processes 3 times, the supernatant was removed and 100 µL of borate buffer (10 mM) was introduced thereinto. A 10 X Avidin-AuNP conjugate solution was prepared as a final suspension solution by vortexing and spinning down the resulting mixture.

### Example 1: Preparation of lateral flow assay strip

A LFA strip consists of four components: a sample pad, a conjugate pad, a nitrocellulose membrane, and an absorption pad. The components are secured on a backing card with polyester. The backing card was cut into 4 mm width using a cutter to prepare an LFA strip. An avidin (1 mg/mL) was loaded on a test line on the nitrocellulose membrane, and biotin-BSA (1 mg/mL) was loaded on a control line on the nitrocellulose membrane, by using a pipette of 1 µL. The distance between these two lines was 3 mm. After loading, the nitrocellulose membrane was dried at 37°C for 1 hour. A 2X Avidin-AuNP conjugate was loaded on the conjugate pad (4 mm X 8 mm) and stored after drying at a temperature of 37°C and 25% humidity. Thereafter, the sample pad, the conjugate pad, the nitrocellulose membrane, and the absorption pad were assembled on an adhesive backing card in this sequence. Every segment overlapped each other by 1.5 mm to facilitate solution movement during the analysis process.

### Example 2: RT-LAMP in a presence of influenza virus RNA

### Example 2-1

To amplify influenza virus RNA through Reverse Transcription Loop-mediated Isothermal Amplification (RT-LAMP) reaction, 10X isothermal buffer (NEB), dNTP (0.3 mM), Biotin-dUTP (0.01 mM), Betaine (0.8M), 6 primers (1.6 µM FIP, BIP/ 0.2 µM F3, B3/ 0.4 µM LB, LF), Bst polymerase (8U), and an aqueous solution containing 10 ng of influenza virus RNA were mixed to make a final volume of 25 µL, followed by performing a reaction at 65°C for 30 minutes.

In this case, FIP stands for a Forward Internal Primer, BIP stands for a Backward Internal Primer, F3 stands for a Forward outer primer, B3 stands for a Backward outer primer, LB stands for a Backward loop primer, and LF stands for a Forward loop primer.

### Example 2-2

Influenza virus RNA was amplified in the same manner as in Example 2-1, except that Biotin-dUTP (0.02 mM) was mixed instead of Biotin-dUTP (0.01 mM).

### Example 2-3

Influenza virus RNA was amplified in the same manner as in Example 2-1, except that Biotin-dUTP (0.04 mM) was mixed instead of Biotin-dUTP (0.01 mM).

### Example 2-4

Influenza virus RNA was amplified in the same manner as in Example 2-2, except that an aqueous solution in which 1 ng of influenza virus RNA was dissolved was mixed instead of an aqueous solution in which 10 ng of influenza virus RNA was dissolved.

### Example 2-5

Influenza virus RNA was amplified in the same manner as in Example 2-2, except that an aqueous solution in which 0.1 ng of influenza virus RNA was dissolved was mixed instead of an aqueous solution in which 10 ng of influenza virus RNA was dissolved.

### Example 3: RT-LAMP in an absence of influenza virus RNA

### Example 3-1

10X isothermal buffer (NEB), dNTP (0.3 mM), Biotin-dUTP (0.01 mM), Betaine (0.8M), 6 primers (1.6 µM FIP, BIP/ 0.2 µM F3, B3/ 0.4 µM LB, LF) and Bst polymerase (8U) were mixed to make a final volume of 25 µL, and this mixture was amplified at 65°C for 30 minutes, but amplification was not proceeded due to an absence of influenza virus RNA.

### Example 3-2

An amplification was proceeded in the same manner as in Example 2-1, except that Biotin-dUTP (0.02 mM) was mixed instead of Biotin-dUTP (0.01 mM), but the amplification was not proceeded because influenza virus RNA was not present.

### Example 3-3

An amplification was proceeded in the same manner as in Example 2-1, except that Biotin-dUTP (0.04 mM) was mixed instead of Biotin-dUTP (0.01 mM), but the amplification was not proceeded because influenza virus RNA was not present.

Amounts of Biotin-dUTP and influenza virus RNA of Examples 2-1 to 2-5 and Examples 3-1 to 3-3 are summarized in Table 1 below.

**Table 1.**

| | Concentration of Biotin-dUTP (mM) | Amount of Influenza virus RNA (ng) |
|---|---|---|
| Example 2-1 | 0.01 | 10 |
| Example 2-2 | 0.02 | 10 |
| Example 2-3 | 0.04 | 10 |
| Example 2-4 | 0.02 | 1 |
| Example 2-5 | 0.02 | 0.1 |
| Example 3-1 | 0.01 | - |
| Example 3-1 | 0.02 | - |
| Example 3-3 | 0.04 | - |

### [Experimental example]

### Experimental example 1: Test line assay for biotin count using synthetic oligomers

Synthetic oligomer having six biotins tagged [Biotin-TEG]AAAAAAAAAA[Biotin-TEG]AAAAAAAAAA[Biotin-TEG] AAAAAAAAAA [Biotin-TEG]AAAAAAAAAA[Biotin-TEG]AAAAAAAAAA[Biotin-TEG] and synthetic oligomer having one biotin tagged [Biotin-TAG]AAAAAAAAAAAAAAAAAAAAAAAAAAAAAAAAAAAAAAAAAAAAAAAAAA were manufactured and synthesized by Bioneer Co., Ltd., an oligomer synthesis company, according to a "phosphite triester" method, in which phosphodiester bonds in a backbone of DNA structure are linked by cyanoethyl phosphoramidite developed by Koster. The synthesis process starts from the nucleoside-attached solid support and repeats cycles consisting of deblocking, coupling, oxidation, and capping to obtain an oligonucleotide of a desired base sequence.

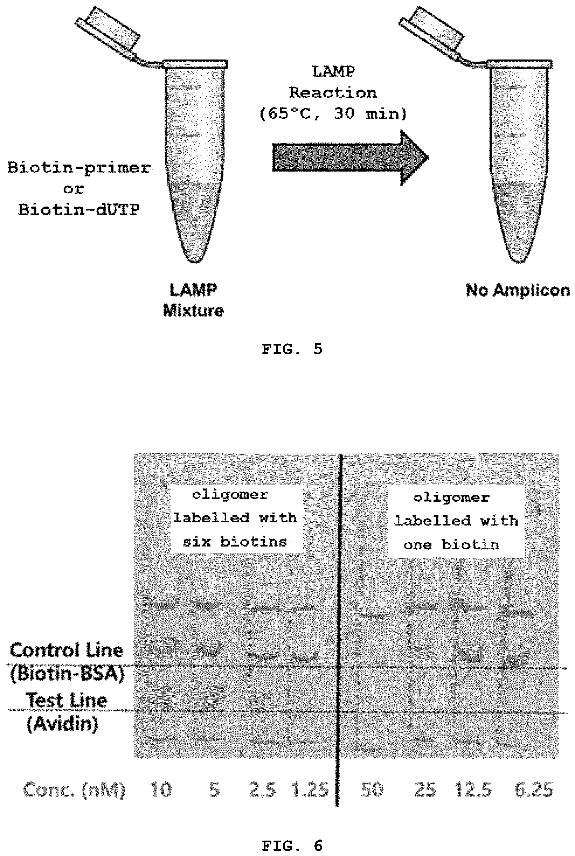

Each of 10 nM, 5 nM, 2.5 nM, and 1.25 nM of the synthetic oligomers having six biotins tagged, and each of 50 nM, 25 nM, 12.5 nM, and 6.25 nM of the synthetic oligomers having one biotin tagged were introduced into LFA strip prepared in Example 1, and then LFA test was performed. Results are shown in FIG. 6. In FIG. 6, biotin-BSA bound to a protein was used instead of "BSA", bovine serum albumin because it is difficult to immobilize small biotin molecules on the NC membrane. Referring to FIG. 6, color change might have been confirmed in a test line of the LFA strip only in the case of synthetic oligomers having six biotins tagged.

### Experimental example 2: Assay of amplification results

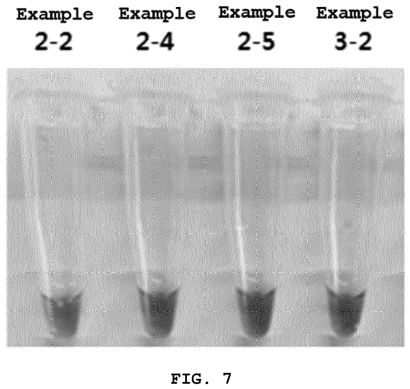

Amplification results obtained from Examples 2-2, 2-4, 2-5 and Example 3-2 are shown in FIG. 7. Referring to FIG. 7, it was confirmed that the color change from purple to light blue occurred in the presence of influenza virus RNA.

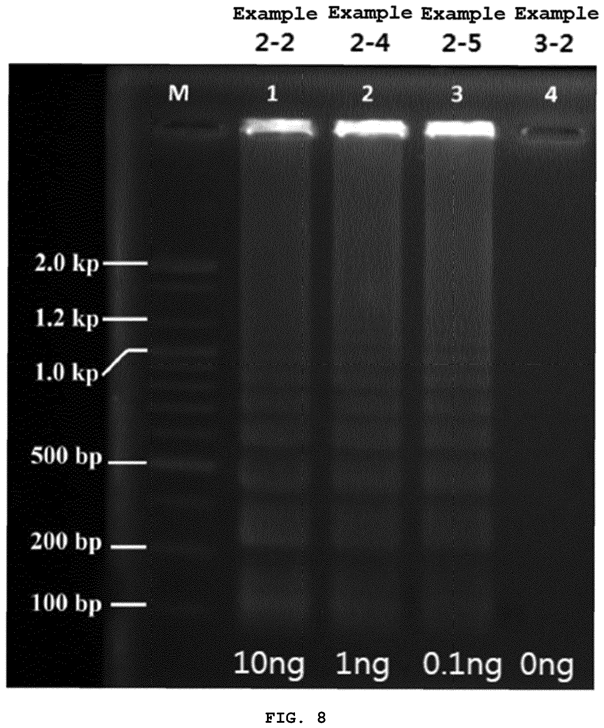

Results of electrophoresis of amplification products obtained from Examples 2-2, 2-4, 2-5 and Example 3-2 are shown in FIG. 8. Referring to FIG. 8, it was confirmed a ladder-shaped pattern when influenza virus RNA was present.

### Experimental example 3: Lateral flow assay depending on a concentration of biotin-dUTP

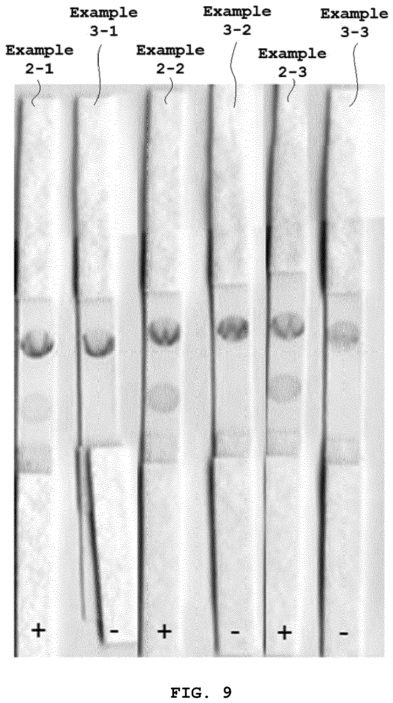

A lateral flow assay was performed by introducing amplification products obtained from Examples 2-1 to 2-3 and Examples 3-1 to 3-3, into a LFA strip obtained from Example 1, and results are shown in FIG. 9. In this case, lateral flow assay was performed by using 150 µL of running buffer (1.0M GH) and 1.0 µL of amplicon, and a reaction time was 10 minutes. In FIG. 9, "+" means that nucleic acid is present, "-" means that nucleic acid is not present.

Referring to FIG. 9, it was confirmed that a signal was weakened when a biotin concentration was lowered. When a concentration of Biotin-dUTP was reduced, biotins of all amplicons bind to avidins, a detector bound to a conjugate, whereby biotins to be bound to the avidins in a test line disappear, thereby weakening the signal. This can be solved through optimization of a concentration of an amplicon precursor which is labelled with the biotin, and a concentration of the avidin coated on the conjugate.

### Experimental example 4: lateral flow assay using LAMP amplicon

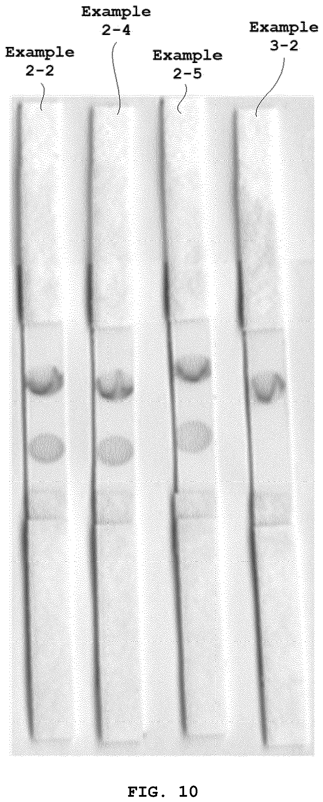

A lateral flow assay was performed by introducing the amplification products obtained from Examples 2-2, 2-4 and 2-5 and Examples 3-2, into the LFA strip prepared in Example 1, and results are shown in FIG. 10. Referring to FIG. 10, color change was confirmed in a test line of a LFA strip only when influenza virus RNA was present.

In the above, although preferred embodiments of the present disclosure have been described, the present disclosure may be variously modified and changed by adding, changing, or deleting components of the present disclosure without departing from a spirit of the present disclosure specified in claims, and this will also be included within the scope of the present disclosure. For example, each component described as a single type may be implemented in a separate form, and likewise components described in a separate form may be implemented in a combined form. The scope of the present disclosure is indicated by the following claims rather than the above detailed description, and all changes or modifications derived from the meaning and scope of the claims and their equivalents should be interpreted as being included in the scope of the present disclosure.

### Industrial Applicability

A lateral flow assay strip of the present disclosure can detect amplicons such as nucleic acids amplified by using a primer or an amplicon precursor such as dNTP, on which one type of tag is labelled, and has an effect of confirming result immediately with naked eyes.

In addition, a lateral flow assay strip has an effect of having high sensitivity due to using at least one tag which has a strong binding force, a trapping molecule which binds the tag(s), a detector, and a trapping molecule which binds to the detector.

In addition, there is a reproducible and stable effect due to using at least one tag which has a constant binding force, a trapping molecule which binds to the tag(s), a detector, and a trapping molecule which binds to the detector.

## Claims

1. A lateral flow assay strip (10) comprising:
a sample pad (100) configured to receive a sample containing at least one selected from an amplicon labelled with at least one tag and an amplicon precursor labelled with a tag;
a conjugate pad (200) comprising a first conjugate (C1) having an indicator and a detector bound to the indicator, with the first conjugate (C1) attached in a flowable manner;
a test pad (300) comprising a test line with a first trapping molecule fixed;
a control pad (400) comprising a control line with a second trapping molecule fixed; and
an absorption pad (500).

2. The lateral flow assay strip of claim 1, wherein the detector is the same type as the first trapping molecule.

3. The lateral flow assay strip of claim 2, wherein each of the detector and the first trapping molecule comprises one selected from the group consisting of an avidin and an anti-biotin antibody.

4. The lateral flow assay strip of claim 1, wherein the indicator comprises one selected from the group consisting of gold (Au), silver (Ag), copper (Cu), platinum (Pt), palladium (Pd), ruthenium (Ru), titanium (Ti), zirconium (Zr), hafnium (Hf), vanadium (V), niobium (Nb), tantalum (Ta), chromium (Cr), molybdenum (Mo), tungsten (W), osmium (Os), iron (Fe), nickel (Ni), cobalt (Co), magnesium oxide (MgO), titanium dioxide (TiO₂), vanadium pentoxide (V₂O₅), and zinc oxide (ZnO) .

5. The lateral flow assay strip of claim 1, wherein each of at least one tag labelled on the amplicon and the tag labelled on the amplicon precursor is the same type as the second trapping molecule.

6. The lateral flow assay strip of claim 5, wherein each of the tag(s) and the second trapping molecule comprises at least one biotin.

7. The lateral flow assay strip of claim 1, wherein the amplicon labelled with at least one tag is labelled with two or more tags.

8. The lateral flow assay strip of claim 1, wherein:
the sample comprises an amplicon labelled with at least one tag;
the sample pad (100) is configured to receive the sample and to discharge the sample to the conjugate pad (200);
the conjugate pad (200) is configured to bind at least one tag labelled on the amplicon to a portion of the first conjugate (C1), so that the first conjugate (C1) and a second conjugate (C2) bound to at least one tag labelled on the amplicon are discharged to the test pad (300);
the test pad (300) is configured to bind the second conjugate (C2) that is bound to at least one tag labelled on the amplicon discharged from the conjugate pad (200), to the first trapping molecule on the test line (310) to trap the second conjugate (C2), and configured to discharge the first conjugate (C1) to the control pad 400; and
the control pad (400) is configured to bind the first conjugate (C1) discharged from the test pad (300), to the second trapping molecule on the control line (400) to trap the first conjugate (C1).

9. The lateral flow assay strip of claim 1, wherein:
the sample comprises an amplicon precursor labelled with a tag;
the sample pad (100) is configured to receive the sample and to discharge the sample to the conjugate pad (200);
the conjugate pad (200) is configured to bind at least one tag labelled on the amplicon to a portion of the first conjugate (C1) to discharge the first conjugate (C1) and a conjugate (C3) that is bound to the tag labelled on the amplicon precursor to the test pad (300);
the test pad (300) is configured to discharge the first conjugate (C1) discharged from the conjugate pad (200) and the conjugate (C3) bound to the tag labelled on the amplicon precursor, to the control pad (400);
the control pad (400) is configured to bind the first conjugate (C1) discharged from the test pad (300) to the second trapping molecule on the control line (410) to trap the first conjugate (C1), and is configured to discharge the third conjugate (C3) bound to the tag labelled on the amplicon precursor to the absorption pad (500); and
the absorption pad (500) is configured to absorb the conjugate (C3) bound to the tag labelled on the amplicon precursor.

10. The lateral flow assay strip of claim 1, wherein the amplicon precursor comprises at least one selected from the group consisting of dNTP, dUTP, a forward inner primer (FIP), a backward inner primer (BIP), a forward outer primer (F3), a backward outer primer (B3), a backward loop primer (LB), and a forward loop primer (LF).

11. The lateral flow assay strip of claim 1, wherein the amplicon labelled with at least one tag is obtained through loop mediated isothermal amplification (LAMP).

12. The lateral flow assay strip of claim 1, wherein the lateral flow assay strip is used to diagnose at least one selected from among bacterial pneumonia, tuberculosis, gonorrhea, and diseases caused by at least one virus selected from the group consisting of influenza virus, AIDS virus (HIV), variola virus, foot-and-mouth disease virus, Ebola virus, dengue virus, Zika virus, corona virus, and HPV virus.

13. The lateral flow assay strip of claim 1, wherein the sample pad (100), the conjugate pad (200), the control pad (300), and the absorption pad (400) are arranged in this order in a horizontal direction, and
the test pad (300) is disposed between the conjugate pad 200 and the control pad (400) or between the control pad (400) and the absorption pad (500).

14. A molecular diagnostic method using a lateral flow assay strip (10) comprising a sample pad (100), a conjugate pad (200), a test pad (300), a control pad (400), and an absorption pad (400), the method comprising steps of:
(a) introducing a sample containing an amplicon labelled with at least one tag, into the sample pad (100);
(b) by the sample pad (100), discharging the sample to the conjugate pad (200);
(c) by the conjugate pad (200), binding at least one tag labelled on the amplicon to a portion of a first conjugate (C1) and discharging the first conjugate (C1) and a second conjugate (C2) bound to at least one tag labelled on the amplicon, to the test pad (300), wherein the conjugate pad 200 comprises the first conjugate (C1) having an indicator and a detector bound to a surface of the indicator, with the first conjugate (C1) attached in a flowable manner;
(d) by the test pad (300), binding the second conjugate (C2) bound to at least one tag labelled on the amplicon discharged from the conjugate pad (200) to a first trapping molecule on a test line to trap the second conjugate (C2) and discharging the first conjugate (C1) that is not trapped by the first trapping molecule to the control pad (400), wherein the test pad (300) comprises the test line having the first trapping molecule fixed; and
(e) by the control pad (400), binding the first conjugate (C1) discharged from the test pad (300) to a second trapping molecule on a control line (410) to trap the first conjugate (C1), wherein the control pad (400) comprises the control line (410) having the second trapping molecule fixed.

15. The method of claim 14, further comprising a step of:
(a') amplifying a mixture containing a nucleic acid, an amplicon precursor labelled with a tag, and an amplicon precursor not labelled with any tag, to obtain an amplicon labelled with a tag or a plurality of tags, before step (a), wherein the amplicon precursor comprises at least one selected from the group consisting of dNTP, dUTP, a forward inner primer (FIP), a backward inner primer (BIP), a forward outer primer (F3), a backward outer primer (B3), a backward loop primer (LB), and a forward loop primer (LF).

16. The method of claim 15, wherein a ratio C2/C1 of a concentration C2 of the amplicon precursor labelled with a tag in the mixture to a concentration C1 of the amplicon precursor not labelled with any tag, used in step (a'), is 0.01 to 0.5.

17. The method of claim 15, wherein the amplification is performed by loop mediated isothermal amplification (LAMP), and the loop mediated isothermal amplification is performed by maintaining a predetermined temperature using at least one selected from the group consisting of a hot pack, a hand warmer, a portable tumbler, and a portable heating device.

18. The method of claim 14, further comprising a step of:
(f) confirming whether each of the test line and the control line generates a colored band to make diagnosis, after step (e).

19. A molecular diagnostic method using a lateral flow assay strip (10) comprising a sample pad (100), a conjugate pad (200), a test pad (300), a control pad (400), and an absorption pad (500), the method comprising steps of:
introducing a sample containing an amplicon precursor labelled with a tag, into the sample pad (100) (step 1);
by the sample pad (100), discharging the sample to the conjugate pad (200) (step 2);
by the conjugate pad (200), binding the tag labelled on the amplicon precursor to a portion of a first conjugate (C1) and discharging the first conjugate (C1) and a third conjugate (C3) bound to the tag labelled on the amplicon precursor, to the test pad (300), wherein the conjugate pad (200) comprises the first conjugate (C1) having an indicator and a detector bound to a surface of the indicator, with the first conjugate (C1) bound in a flowable manner (step 3);
by the test pad (300), discharging the first conjugate (C1) discharged from the conjugate pad (200) and the third conjugate (C3) bound to the tag labelled on the amplicon precursor, to the control pad (400), wherein the test pad (300) comprises a test line having a first trapping molecule fixed (step 4);
by the control pad (400), binding the first conjugate (C1) discharged from the test pad (300) to a second trapping molecule on a control line to trap the first conjugate (C1) and discharging the third conjugate (C3) bound to the tag labelled on the amplicon precursor, to the absorption pad (500), wherein the control pad (400) comprises the control line having the second trapping molecule fixed (step 5); and
by the adsorption pad (500), absorbing the third conjugate (C3) bound to the tag labelled on the amplicon precursor (step 6) .

20. The method of claim 19, further comprising a step of:
amplifying a mixture containing an amplicon precursor which is labelled with a tag and an amplicon precursor which is not labelled with any tag (step 1'), before the step 1, wherein the amplification results are not obtained.
